(19) Europäisches Patentamt
European Patent Office
Office européen des brevets

(11) Veröffentlichungsnummer : **0 016 956**
**B1**

(12) # EUROPÄISCHE PATENTSCHRIFT

(45) Veröffentlichungstag der Patentschrift : **07.04.82**

(21) Anmeldenummer : **80100905.1**

(22) Anmeldetag : **25.02.80**

(51) Int. Cl.³ : **C 07 D249/12**// C08G63/68

(54) **Neue Trishydroxyalkylheterocyclen und Verfahren zu ihrer Herstellung.**

(30) Priorität : **06.03.79 DE 2908627**

(43) Veröffentlichungstag der Anmeldung : **15.10.80 (Patentblatt 80/21)**

(45) Bekanntmachung des Hinweises auf die Patenterteilung : **07.04.82 Patentblatt 82/14**

(84) Benannte Vertragsstaaten : **AT BE DE FR GB IT NL SE**

(56) Entgegenhaltungen : **GB - A - 1 290 729**

(73) Patentinhaber : **BAYER AG**
**Zentralbereich Patente, Marken und Lizenzen**
**D-5090 Leverkusen 1, Bayerwerk (DE)**

(72) Erfinder : **Merten, Rudolf, Dr.**
**Berta-von-Suttner-Strasse 55**
**D-5090 Leverkusen (DE)**
Erfinder : **Rottmaier, Ludwig. Ing. grad.**
**Bergstrasse 85**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Imprimerie Jouve, 18, rue St-Denis, 75001 Paris, France

Neue Trishydroxyalkylheterocyclen und Verfahren zu ihrer Herstellung

Die vorliegende Erfindung betrifft neue N-heterocyclische Triole der allgemeinen Formel

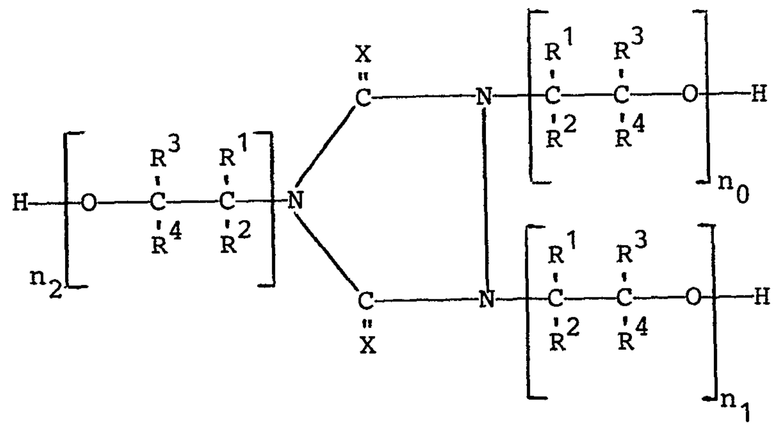

(I)

worin

$n_0$, $n_1$ und $n_2$ gleich oder verschieden für eine ganze Zahl von 1 bis 30, vorzugsweise 1-10,

X, gleich oder verschieden, für O oder S,

$R^1$, $R^2$, $R^3$ und $R^4$ gleich oder verschieden, unabhängig voneinander für Wasserstoff, einen gegebenenfalls mit Halogen substituierten, aliphatischen $C_1$-$C_{10}$, cycloaliphatischen $C_4$-$C_8$ oder araliphatischen $C_7$-$C_{17}$ Rest oder einen gegebenenfalls mit Halogen, Alkyl und/oder Alkoxy substituierten aromatischen $C_6$-$C_{16}$ Rest stehen. Vorzugsweise bedeuten $R^1$, $R^2$, $R^3$ und $R^4$ Wasserstoff, einen gegebenenfalls mit Halogen (Chlor, Brom) substituierten Alkylrest mit $C_1$-$C_4$, vorzugsweise Methyl, Ethyl oder einen gegebenenfalls mit Halogenatomen (Chlor, Brom), $C_1$-$C_4$-Alkylresten und/oder mit $C_1$-$C_4$ Alkoxygruppen substituierten $C_6$-$C_{12}$ Arylrest, vorzugsweise Phenyl.

Besonders bevorzugt leiten sich die Reste von Ethylen- und Propylenoxid-Einheiten mit einem Oxalkylierungsgrad von $n = 1$ ab.

Die erfindungsgemäßen Triole der allgemeinen Formel I sind neu und werden erhalten, indem man Triazolidindion-3,5 (= Uracol) mit Alkylenoxiden, vorzugsweise Ethylenoxid, Propylenoxid, Butylenoxid, Styroloxid gegebenenfalls in Gegenwart eines geeigneten Katalysators umsetzt. Es können jedoch auch die Thionanalogen des Uracols eingesetzt werden. Die Addition des Alkylenoxids an die drei NH-Gruppen des Triazolidindions-3,5 kann sowohl bei Anwesenheit von sauren als auch alkalischen Katalysatoren vorgenommen werden. Vorzugsweise verwendet man jedoch bei der Herstellung der Triole der Formel I basische Katalysatoren wie Tetraethylammoniumchlorid, tertiäre Amine wie Triethylamin und Dimethylanilin und Alkali- oder Erdalkalihydroxide oder deren Carbonate wie Calciumhydroxid oder Kaliumcarbonat. Es können jedoch auch Alkalihalogenide wie Lithiumchlorid eingesetzt werden. Die Menge an Katalysator liegt zwischen 0,05 und 3 %, bezogen auf die Reaktanten.

Eine bevorzugte Ausführungsform ist die Addition von drei Mol Ethylenoxid an ein Mol Uracol ohne Verwendung eines Katalysators, wobei eine praktisch quantitative Bildung von N,N',N''-Tris-hydroxyethyluracol erfolgt. Diese Reaktion ist auch deshalb überraschend, da die drei NH aus dem Uracol aufgrund ihrer unterschiedlichen Basizität eine unterschiedliche Reaktivität aufweisen sollten, wonach wiederum eine Addition des Ethylenoxids an bereits vorhandene Hydroxylgruppen und somit eine Bildung von Polyether-Struktureinheiten zu erwarten wäre. Die Addition des Ethylenoxids an das Uracol kann bei Abwesenheit eines Katalysators sehr gut verfolgt werden, da nach dem Verbrauch von 3 Mol Ethylenoxid unter Normaldruck kaum weiteres Ethylenoxid aufgenommen wird. Die Herstellung der Trishydroxyverbindungen gemäß Formel I erfolgt vorzugsweise unter Einsatz äquivalenter Mengen, d.h. pro Mol Uracol werden 3 $(n_0 + n_1 + n_2)$ Mole Alkylenoxid eingesetzt.

Das zur Herstellung der neuen Triole der Formel I verwendete Triazolidindion-3,5 ist aus Liebigs Annalen der Chemie, Band 283, Seite 41 (1894) bekannt und kann z.B. durch Erhitzen auf über 200 °C aus Hydrazin-N,N'-dicarbonsäurediamid unter Ammoniakabspaltung erhalten werden.

Die Reaktion des Uracols mit dem Alkylenoxid wird vorzugsweise in inerten organischen Lösungsmitteln durchgeführt. Insbesondere geeignet sind polare organische Lösungsmittel wie Dimethylsulfoxid, Tetramethylensulfon, Dimethylformamid, Dimethylacetamid und N-Methylpyrrolidion-2. Wasser ist auch sehr gut geeignet als Lösungsmittel, wobei überraschenderweise keine Reaktion des Ethylenoxids mit dem Wasser zu beobachten ist. Dabei ist es nicht unbedingt erforderlich, das Uracol gelöst mit dem Alkylenoxid in Reaktion zu bringen. Die Reaktion kann auch mit einer Uracol-Suspension erfolgen, wobei das gebildete Triol dann in Lösung geht, so daß das Ende der Reaktion durch eine klare Lösung angezeigt wird. Auch kann in die entstehende Triollösung Uracol und Alkylenoxid gemeinsam zugesetzt werden, so daß die Lösungsmittelmenge sehr niedrig gehalten wird. Die Menge an Lösungsmittel sollte

aus wirtschaftlichen Gründen sehr niedrig sein und kann zwischen 0,3 Gew.-Teilen und 20 Gew.-Teilen Lösungsmittel, bezogen auf ein Gew.-Teil Reaktanten liegen. Nach beendeter Reaktion wird das Lösungsmittel durch Anlegen von Vakuum entfernt und der verbleibende, viskose Rückstand durch übliche Arbeitsmethoden wie Umkristallisieren gereinigt. Häufig kann jedoch auf eine Reinigung verzichtet werden und das Rohprodukt wird sofort weiterverarbeitet.

Die Reaktion wird vorzugsweise bei Temperaturen von 25 °C bis 200 °C, besonders bevorzugt bei 80 °C bis 150 °C durchgeführt.

Die Reaktionszeiten liegen im allgemeinen zwischen 30 Minuten und mehreren Tagen, können in besonderen Fällen jedoch auch darüber oder darunter liegen. Durch entsprechende Wahl der Reaktionsbedingungen, z.B. Druck, werden kürzere Reaktionszeiten erreicht.

Eine besonders bevorzugte Ausführungsform ist die Umsetzung von Uracol in einem niederen Dialkylformamid (z.B. Dimethylformamid) mit Ethylenoxid bei einer Temperatur von 120 °C. Das Lösungsmittel wird nach der Reaktion im Wasserstrahlvakuum entfernt und dann bei 0,2 mbar und 90-100 °C bis zur Gewichtskonstanz eingeengt. Der entstehende viskose Rückstand kristallisiert sehr schnell durch und kann nach Aufschlämmen in Ethanol oder Isopropanol abgesaugt werden. Das erhaltene nahezu reine Tris-2-hydroxyethyl-triazolidindion-3,5 kann durch Umkristallisation in Aceton oder Isopropanol noch weiter gereinigt werden.

Die Tris-2-hydroxyalkyl-triazolidindione der Formel I sind wertvolle Ausgangsprodukte zur Herstellung von polymeren Verbindungen. Sie können als Vernetzer in hydroxylgruppenhaltige bekannte Polyester aus z.B. Adipinsäure, Phthalsäure, Diglykol eingebaut werden und werden zur Herstellung von Hart- oder Weichschaum-Polyurethanen verwendet.

Die erfindungsgemäßen Verbindungen der Formel I, insbesondere wenn n = 1 ist, können als Starter zur Herstellung von hydroxylgruppenhaltigen Polyethern und die wiederum je nach Molgewicht, zur Herstellung von Hart- oder Weichschaum-Polyurethanen verwendet werden.

## Beispiel 1

In einem 500 ml Dreihalskolben, der mit Rührer, Thermometer und einem Wasser gekühlten Rückflußkühler versehen ist, werden 50,5 g (0,5 Mol) Uracol in 150 ml Dimethylformamid gelöst. In diese Lösung werden bei 120 °C 66 g (1,5 Mol) Ethylenoxid so eingeleitet, daß durch einen auf dem Rückflußkühler aufgesetzten Blasenzähler kein Ethylenoxid entweicht. Nach Beendigung der Reaktion wird in Vakuum, zuletzt bei 0,2 mbar/100 °C bis zur Gewichtskonstanz eingeengt. Es werden so 117 g einer viskosen Flüssigkeit erhalten, die lt. Gaschromatogramm über 85 % des reinen Tris-hydroxyethyltriazolidin-dion-3,5 enthält, welches nach Stehenlassen des viskosen Rückstandes allmählich auskristallisiert. Das reine Tris-hydroxyethyl-triazolidindion-3,5 vom Fp = 90 °C erhält man auch, indem das viskose Rohprodukt in Isopropanol gelöst und druch Abkühlen zur Kristallisation gebracht wird. IR- und NMR-Spektren sowie die Elementaranalyse stimmen mit der angenommenen Struktur überein.

| | | | | |
|---|---|---|---|---|
| Ber.: | 41,2 % C | 6,4 % H | 18 % N | 21,9 % OH |
| Gef.: | 41,4 % C | 6,5 % H | 17,9 % N | 21,7 % OH |

## Beispiel 2

In einem Druckkessel werden zu einer Mischung aus 303 g (3 Mol) Uracol, 30 g pulverisiertem Ätznatron und 450 ml Dimethylformamid bei 105 °C 1 000 g Propylenoxid so zudosiert, daß der Innendruck nicht über 8 bar steigt. Nach Beendigung der Reaktion werden im Vakuum, zuletzt bei 0,2 mbar und 100 °C, die flüchtigen Bestandteile entfernt. Der Rückstand wird in Chloroform gelöst, mit verd. $H_2SO_4$ neutralisiert, das abgeschiedene Natriumsulfat abfiltriert und im Vakuum das Lösungsmittel bis zur Gewichtskonstanz entfernt. Es werden 1 272 g (= 97,7 % Ausbeute) eines gelblichen, öligen Polyäthers mit einer Viskosität $\eta$ 25 = 8 692 mPa·s erhalten, dessen IR-Spektrum mit der angenommenen Struktur übereinstimmt. Die Analyse ergab einen Hydroxylgehalt von 12 % (berechnet: 12,1 %) und einen Stickstoffgehalt von 9,8 % (berechnet 9,9 %).

## Beispiel 3

50,5 g (0,5 Mol) Uracol werden in einem Dreihalskolben, der mit Rührer, Thermometer und Rückflußkühler ausgestattet ist, in 150 ml Dimethylformamid gelöst und mit 2,5 g Tetraethylammoniumchlorid versetzt. In diese Mischung werden bei 120 °C 108 g iso-Butylenoxid zugetropft und 2 Stunden bei 120 °C nachgerührt. Nach dem Entfernen der flüchtigen Bestandteile im Vakuum, zuletzt bei 0,2 mbar und 100 °C, werden 158 g eines hellgelben, viskosen Rückstandes erhalten, dessen IR-Spektrum mit der angenommenen Struktur übereinstimmt. Die Analyse ergab einen Hydroxylgehalt von 15,9 % OH (berechnet 16,1 % OH) während die Elementaranalyse folgende Werte ergab:

| | | | |
|---|---|---|---|
| berechnet: | 53,0 % C | 8,52 % H | 13,25 % N |
| gefunden: | 52,8 % C | 8,44 % H | 13,5 % N |

## Beispiel 4

50,5 g (0,5 mol) Uracol werden in einem Dreihalskolben, der mit Rührer, Thermometer und Rückflußkühler ausgestattet ist, in 150 g Wasser gelöst. Nach Zugabe von 0,5 g Endoethylenpiperazin werden unter Rückfluß des Lösungsmittels 66 g (1,5 mol) Ethylenoxid so eingeleitet, daß kein Ethylenoxid entweicht. Nach Beendigung der Reaktion wird im Vakuum, zuletzt bei 0,2 mbar/80 °C bis zur Gewichtskonstanz eingeengt. Es werden so 115 g einer viskosen Flüssigkeit erhalten, die lt. Gaschromatogramm ca. 75 % des reinen Tris-hydroxyethyl-triazolidindion-3,5 enthält.

## Beispiel 5

Analog Beispiel 4 werden 50,5 g Uracol in 150 g Dimethylformamid gelöst, mit 0,75 g Lithiumchlorid versetzt und mit 66 g Ethylenoxid umgesetzt. Nach dem Einengen werden 114 g einer viskosen Flüssigkeit erhalten, die nach einigen Stunden zu kritallisieren beginnt und die lt. Gaschromatogramm 82 % an reiner Tris-hydroxyethyltriazolidindion-3,5 enthält.

## Beispiel 6

In einem Dreihalskolben, der mit Rührer, Thermometer und einem mit Trockeneis/Methanol-Gemisch gekühlten Rückflußkühler versehen ist, werden 1 010 g (10 mol) Uracol in 1 000 g Dimethylformamid suspendiert und mit 30 g Triethylamin versetzt.

Bei 120 °C werden dann insgesamt 1 320 g Ethylenoxid so eingeleitet, daß sich leichter Rückfluß einstellt und daß die zwischenzeitlich stark exotherme Reaktion durch Luftkühlung auf 120 °C gehalten werden kann. Nach beendeter Reaktion wird im Vakuum zuletzt bei 0,2 mbar und 80 °C bis zur Gewichtskonstanz eingeengt. Es wurden 2 330 g einer viskosen Lösung erhalten, die anderntags durchkristallisiert war und die nach gaschromatographischer Analyse 85 % an reinem Tris-2-hydroxyethyl-triazolidindion-3,5 enthält.

## Beispiel 7

505 g Uracol wurden in einem Dreihalskolben (Versuchsanordnung analog Beispiel 6) in 1 500 g Tetramethylensulfon suspensiert und mit 10 g Tetraethylammoniumchlorid versetzt. Durch Einleiten von Ethylenoxid bei 120 °C entstand eine klare Lösung. Dann wurden nochmals 303 g Uracol suspendiert und sobald wieder eine klare Lösung entstanden war, kamen nochmals 202 g Uracol dazu. Nachdem insgesamt 1 310 g Ethylenoxid eingeleitet waren, wurde im Vakuum, zuletzt bei 0,2 mbar und 120 °C, eingeengt. Es wurden so 2 354 g einer viskosen Flüssigkeit erhalten, die lt. gaschromatographischer Analyse 84,5 % an reinem Tris-2-hydroxyethyl-triazolidindion-3,5 enthält.

Durch Auflösen der viskosen Flüssigkeit in einem Gemisch aus Isopropanol und Aceton und nachfolgendem Abkühlen kann eine kristalline Substanz vom Schmelzpunkt 86-88 °C erhalten werden, welche durch Umkristallisieren aus z.B. Isopropanol einen Schmelzpunkt von 90 °C erreicht und die lt. IR-Spektrum mit dem reinen Tris-2-hydroxyethyl-triazolidindion-3,5 aus Beispiel 1 übereinstimmt.

**Ansprüche**

1. Trishydroxyalkylheterocyclen der allgemeinen Formel I

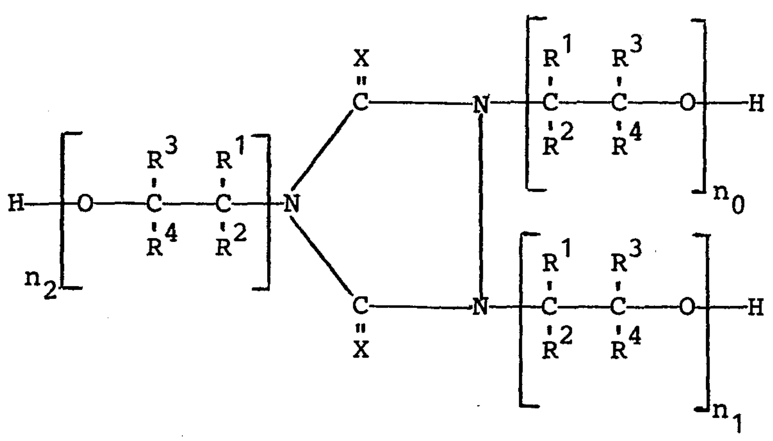

(I)

worin

$n_0$, $n_1$ und $n_2$, gleich oder verschieden für eine Zahl von 1 bis 30,
X, gleich oder verschieden, für O oder S,
$R^1$, $R^2$, $R^3$ und $R^4$, gleich oder verschieden, unabhängig voneinander für Wasserstoff, einen gegebenenfalls mit Halogen substituierten, aliphatischen $C_1$-$C_{10}$, cycloaliphatischen $C_4$-$C_8$ oder araliphatischen $C_7$-$C_{17}$ Rest oder einen gegebenenfalls mit Halogen, Alkyl und/oder Alkoxy substituierten aromatischen $C_6$-$C_{16}$ Rest stehen.

2. Tris-hydroxyalkyl-triazolidin-3,5-dione der allgemeinen Formel gemäß Anspruch 1, in der $n_0$, $n_1$ und $n_2$ jeweils 1 bedeuten.

3. Trishydroxyalkyl-triazolidin-3,5-dione der allgemeinen Formel I gemäß Ansprüchen 1 und 2, in der $R^1$, $R^2$, $R^3$ und $R^4$, gleich oder verschieden Wasserstoff, einen gegebenenfalls mit Halogen substituierten Alkylrest mit $C_1$-$C_4$, einen gegebenenfalls mit Halogenatomen, $C_1$-$C_4$-Alkylresten und/oder mit $C_1$-$C_4$ Alkoxygruppen substituierten $C_6$-$C_{12}$ Arylrest, bedeuten.

4. N,N',N''-Tris-hydroxyethyl-triazolidindion-3,5.

5. Verfahren zur Herstellung von Trishydroxyalkylheterocyclen nach Ansprüchen 1 bis 4, dadurch gekennzeichnet, daß Triazolidindion-3,5 bzw. die entsprechenden Thioanalogen mit Alkylenoxiden der allgemeinen Formel II

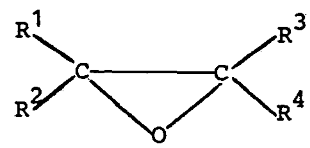

worin
$R^1$, $R^2$, $R^3$ und $R^4$ die in Anspruch 1 angegebene Bedeutung haben,
gegebenenfalls in Gegenwart eines Katalysators und/oder Lösungsmittels umgesetzt werden.

6. Verfahren nach Anspruch 5, dadurch gekennzeichnet, daß pro Mol Triazolidindion-3,5 3 ($n_0 + n_1 + n_2$) Mole Alkylenoxid eingesetzt werden.

7. Verfahren nach Ansprüchen 5 und 6, dadurch gekennzeichnet, daß basische Katalysatoren verwendet werden.

8. Verfahren nach Ansprüchen 5 bis 7, dadurch gekennzeichnet, daß Ethylenoxid verwendet wird.

## Claims

1. Heterocyclic tris-hydroxyalkyl compounds of the general formula I

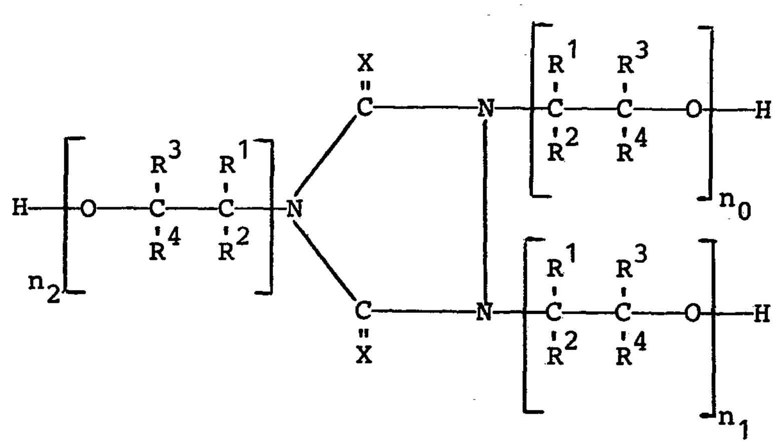

(I)

wherein
$n_0$, $n_1$ and $n_2$ are the same or different and each is an integer of from 1 to 30,
the X's are the same or different and represent O or S,
$R^1$, $R^2$, $R^3$ and $R^4$ are the same or different and independently of one another represent hydrogen, an aliphatic $C_1$-$C_{10}$, cycloaliphatic $C_4$-$C_8$ or araliphatic $C_7$-$C_{17}$ radical optionally substituted by halogen, or an aromatic $C_6$-$C_{16}$ radical optionally substituted by halogen, alkyl and/or alkoxy.

2. Tris-hydroxyalkyl triazolidine-3,5-diones of the general formula according to Claim 1, in which $n_0$, $n_1$ and $n_2$ each denote 1.

3. Tris-hydroxyalkyl triazolidine-3,5-diones of the general formula I according to Claims 1 and 2, in which
$R^1$, $R^2$, $R^3$ and $R^4$ are the same or different and represent hydrogen, an optionally halogen-substituted $C_1$-$C_4$ alkyl radical or a $C_6$-$C_{12}$ aryl radical optionally substituted by halogen atoms, $C_1$-$C_4$

alkyl groups and/or $C_1$-$C_4$ alkoxy groups.

4. N,N',N''-Tris-hydroxyethyl-triazolidine-3,5-dione.

5. Process for producing the heterocyclic tris-hydroxyalkyl compounds according to Claims 1 to 4, characterised in that triazolidine-3,5-dione or the corresponding thio analogues are reacted with alkylene oxides of the general formula II

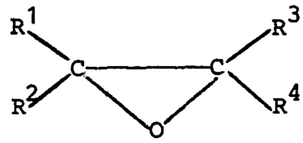

wherein

$R^1$, $R^2$, $R^3$ and $R^4$ have the meaning indicated in Claim 1, optionally in the presence of a catalyst and/or a solvent.

6. Process according to Claim 5, characterised in that 3 ($n_0$ + $n_1$ + $n_2$) moles of alkylene oxide are used per mole of triazolidine-3,5-dione.

7. Process according to Claims 5 and 6, characterised in that basic catalysts are used.

8. Process according to Claims 5 to 7, characterised in that ethylene oxide is used.

**Revendications**

1. Tris-hydroxyalcoyl-hétérocycles de formule générale I

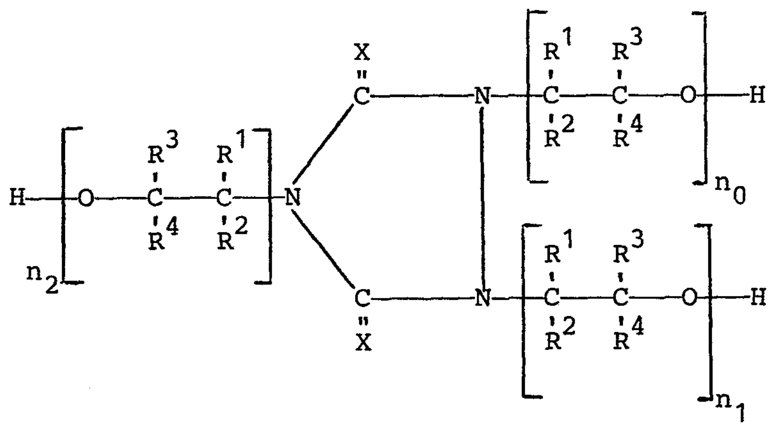

(I)

dans laquelle

$n_0$, $n_1$ et $n_2$ sont identiques ou différents et représentent un nombre de 1 à 30 ;

X sont identiques ou différents et représentent O ou S,

$R^1$, $R^2$, $R^3$ et $R^4$ sont identiques ou différents et représentent indépendamment les uns des autres de l'hydrogène, un radical aliphatique en $C_1$-$C_{10}$, cycloaliphatique en $C_4$-$C_8$ ou araliphatique en $C_7$-$C_{17}$ éventuellement substitué par de l'halogène, ou un radical aromatique en $C_6$-$C_{16}$ éventuellement substitué par de l'halogène, alcoyle et/ou alcoxy.

2. Tris-hydroxyalcoyl-triazolidine-3,5-diones de formule générale selon la revendication 1, dans laquelle

$n_0$, $n_1$ et $n_2$ signifient chacun 1.

3. Tris-hydroxyalcoyl-triazolidine-3,5-diones de formule générale I selon les revendications 1 et 2, dans laquelle

$R^1$, $R^2$, $R^3$ et $R^4$, identiques ou différents, représentent de l'hydrogène, un radical alcoyle en $C_1$-$C_4$ éventuellement substitué par de l'halogène, un radical aryle en $C_6$-$C_{12}$ éventuellement substitué par des atomes d'halogène, des radicaux alcoyle en $C_1$-$C_4$ et/ou des groupes alcoxy en $C_1$-$C_4$.

4. N,N',N''-tris-hydroxyéthyl-triazolidine-dione-3,5.

5. Procédé de préparation de tris-hydroxyalcoyl-hétérocycles selon les revendications 1 à 4, caractérisé en ce qu'on fait réagir de la triazolidine-dione-3,5 ou les thio-analogues correspondants avec des oxydes d'alcoylène de formule générale II

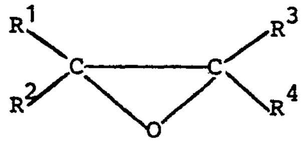

dans laquelle

$R^1$, $R^2$, $R^3$ et $R^4$ ont la signification indiquée à la revendication 1, éventuellement en présence d'un catalyseur et/ou d'un solvant.

6. Procédé selon la revendication 5, caractérisé en ce que par mole de triazolidine-dione-3,5 on utilise $3(n_0 + n_1 + n_2)$ moles d'oxyde d'alcoylène.

7. Procédé selon les revendications 5 et 6, caractérisé en ce qu'on utilise des catalyseurs basiques.

8. Procédé selon les revendications 5 à 7, caractérisé en ce qu'on utilise de l'oxyde d'éthylène.